Europäisches Patentamt

(19) European Patent Office

Office européen des brevets

(11) **EP 0 919 252 A1**

(12) **EUROPEAN PATENT APPLICATION**

(43) Date of publication:
02.06.1999 Bulletin 1999/22

(51) Int. Cl.$^6$: **A61M 15/00**, B05B 12/00

(21) Application number: 97309492.3

(22) Date of filing: 25.11.1997

(84) Designated Contracting States:
AT BE CH DE DK ES FI FR GB GR IE IT LI LU MC
NL PT SE
Designated Extension States:
AL LT LV MK RO SI

(71) Applicant:
THE TECHNOLOGY PARTNERSHIP PUBLIC
LIMITED COMPANY
Melbourn Royston Hertfordshire SG8 6EE (GB)

(72) Inventors:
• Blakey, David Mark
Metcalfe Road, Cambridge CB4 2DX (GB)
• Goodchild, Martin Scott
Cambridge CB4 3QG (GB)

(74) Representative:
Brunner, Michael John
GILL JENNINGS & EVERY
Broadgate House
7 Eldon Street
London EC2M 7LH (GB)

(54) **Aerosol delivery method and apparatus**

(57) A method of and apparatus for providing an aerosol are disclosed in which droplets are sprayed into an evaporation chamber (1) from an aerosol generation device (2) to provide an aerosol. The chamber has an air or gas inlet (13) and an outlet (4) from which the aerosol is withdrawn. The atmosphere within the chamber (1) is controlled in order to control the extent of droplet evaporation within the chamber, in turn to control the size of the aerosol droplets, by sensing the atmosphere within the chamber with a humidity or like sensor (12). The spraying of the droplets into the chamber by the aerosol generator (2) is controlled in dependence upon the output of the sensor (12).

fig. 1

**Description**

The present invention relates to an aerosol droplet apparatus and method of generating an aerosol and, more particularly, to a method and apparatus for delivering droplets of liquid (which may contain particles in the suspension) to the lungs of a patient for diagnosis/examination or treatment purposes.

Methods and apparatus of the above type have many applications in medicine and, for example, are used to deliver drugs, eg. for the treatment of asthma, or for the delivery of materials for examination or diagnostic procedures, eg. for gamma-ray scintigraphy.

Whilst existing nebulizers and other aerosol generation devices are capable of providing droplets of liquid or dispersions of powder in air for various medical uses, the degree of control of the delivery process (including droplet size) is limited and thus, in the case say of nebulizers for the treatment of asthma, there can be wide variations in the amount of drug delivered, from user to user and from use to use. In the case of gamma-ray scintigraphy, for providing lung images, in order to obtain satisfactory images, the doses of radioactive material supplied, may need to be greater than medically desirable in order to allow satisfactory images to be obtained.

These and other problems have led to the desire for greater control of the delivery system in such methods and apparatus and the present invention has, as its object the aim of providing a system in which these problems can be overcome.

According to a first aspect of the present invention therefore there is provided a method of generating an aerosol in which droplets are sprayed into an evaporation chamber to provide an aerosol, the chamber having an air or gas inlet and an outlet from which the aerosol is withdrawn by a user, and in which the atmosphere within the chamber is controlled in order to control the extent of droplet evaporation within the chamber, in turn to control the size of the aerosol droplets, by sensing the atmosphere within the chamber and controlling the spraying of the droplets into the chamber in dependence upon the sensing.

With an aqueous aerosol, controlling the humidity within the chamber allows accurate control of the rate and therefore extent of evaporation so that the size of the droplets or particles within the chamber and which are breathed by the user, can be controlled to a desired size in order to optimise their effect. Similarly, a solvent sensor can be used for non-aqueous aerosols.

The invention also includes a method of controlling the diameter of a liquid droplet aerosol within a chamber, the chamber having an air or gas inlet and an outlet from which the aerosol exits the chamber, wherein the atmosphere within the chamber, the initial droplet diameter, and the droplet residence time are selected in order to control the extent of evaporation within the chamber, in turn to control the size of the aerosol droplets which exit the chamber.

By this means, three sets of control can be provided in operation;

1. Atmospheric conditions (internal and external relative humidity, air or gas temperature, and atmospheric pressure, and air or gas flow rate).
2. Initial droplet diameter (in conjunction with the atmospheric conditions, this defines the rate of droplet evaporation's, and in turn the droplet lifetime). By droplet lifetime we mean the time taken for solvent of the solution or carrier liquid of the suspension to have substantially evaporated.
3. Droplet residence time (this relates to air flow rate, chamber volume, and droplet velocity).

In practice, this allows two preferred modes of operation;

1. The droplets are evaporated to dry (all liquid driven off into the vapour phase). This leaves behind only the non-volatile solids within the solution (or suspension) from which the droplets are initially formed.
2. The droplets are evaporated to a controlled, smaller diameter. In this mode the droplets may be reduced (e.g. from 100μm to 10μm), and remain composed of a liquid solution (although the concentration will be increased by the loss of the solvent through evaporation) or liquid suspension.

These two modes arise naturally from evaporation phenomena.

In the first mode of operation, the atmospheric conditions (of partial vapour pressure (or RH), temperature, atmospheric pressure), air flow rate (which, for a given chamber volume determines mean droplet residence time), and initial droplet diameter are known, so that, for a given suspension or solution, the evaporation rate of a liquid into this atmosphere, and hence the droplet lifetime within this atmosphere is determined. If these conditions are controlled such that the droplet residence time is longer than the droplet lifetime, then the droplets will evaporate to dry. 'Dry' in this context includes the retention by the residual solid particulates of a solvent concentration that is in equilibrium under atmospheric conditions in which the atmosphere has zero partial vapour pressure. For example, if the relative humidity is below 50%RH, and the droplets are generated between ∅5μm and ∅10μm, then the droplet lifetime of water droplets is typically 40ms to 150ms. This represents the shortest droplet residence time needed to dry those droplets within the chamber.

In the second mode of operation, the system conditions (as mentioned above) are known. This time the droplet residence time within chamber 1 is arranged to be shorter than the droplet lifetime, so that the exiting droplets are not fully evaporated. In such a mode of operation, the diameter of the exiting droplets can be determined by the system conditions and the residence time. For example, if the internal humidity is between 70%RH and 100%RH (for example, by fixing the humidity control at a higher level, or using a high humidity air source), or the initial droplet diameter is significantly larger than 10µm, then the droplet lifetime may be extended up to the order of 1s to 100s. In such a system, we can control the final droplet diameter relatively accurately by limiting the residence time. Note that as the droplet diameter decreases, the surface area to volume ratio becomes larger which drives up the evaporation rate, and hence the rate of droplet diameter reduction.

According to a further aspect of the invention there is provided a dosing apparatus comprising a reservoir for containing a material to be delivered to the lungs of a user; an evaporation chamber having an air or gas inlet and an outlet; an aerosol generator for receiving material from the reservoir and for generating an aerosol in the evaporation chamber; an atmosphere sensor within the chamber; and means for controlling the aerosol generator in response to an output of the sensor.

For a given material which is required to be delivered to the user's lungs, the extent of evaporation can thus be controlled by adjusting the humidity through operation of the aerosol generator in order that the droplet or particle size can be controlled to an optimum level.

The usual meaning of 'humidity' relates to water vapour, however the term is used in a more generalised definition according to this specification, referring to any liquid or even solid phase of matter which exhibits a saturated vapour pressure and a measurable partial vapour pressure.

Preferably, the humidity sensor is a resistive or capacitive sensor. Control may, for example, be implemented as a simple ON-OFF control loop by activating the aerosol generator when the sensed humidity inside the evaporation chamber is below a threshold level and deactivating the aerosol generator when the humidity increases above the threshold. Many more sophisticated control methods are also possible and fall within the scope of this invention. Examples are described below.

Aerosol generation may be achieved by a number of different methods and apparatuses, the primary function being to convert a continuous liquid phase material to a discontinuous phase comprising droplets/particles in a gaseous medium, whose diameter is commensurate with the droplet lifetime and inhalation rate from the chamber.

In one embodiment, the aerosol generator comprises an electronic aerosol generating device driven from a self-resonant driver circuit to create droplets having a narrow and small droplet diameter distribution which minimises the need for impact filtering (for example of large droplets). Devices of the type described, for example, in our European Patent 0615470, may be suitable for droplet generation.

Non-aqueous solutions can be evaporated using the same method and apparatus, the sensor comprising a solvent sensor being used to measure the level of solvent saturation in the chamber in order to determine the evaporation rate within the chamber by controlling spraying of the solution.

One example of a method and apparatus according to the present invention will now be described with reference to the accompanying Figure which comprises a diagrammatic representation of the apparatus.

The apparatus shown in the Figure comprises an evaporation chamber 1 (a Volumatic™ Spacer Device manufactured by Allen & Hanburys) on which is mounted an aerosol generator 2 of the type (eg. described in our European Patent 0615470) comprising a piezoelectrically vibrated membrane to which a selected suitable solution is delivered from a reservoir 3 disposed above the chamber 1. The chamber has an air inlet 13 through which, in use, a flow of air is drawn through the chamber 1.

From the bottom of the chamber 1 an outlet 4 extends to a mouthpiece 5 via a tube 6, one-way valve 7 and a T-piece 8 which, in the other 9 branch (open to air) has a further one-way valve 10 and a filter 11. Within the evaporation chamber 1 is mounted a humidity sensor 12 (in the form of an RHU-217-5AT temperature and humidity module from Farnell Mercator or similar) which provides an output in the form of DC voltage normally between 0V and +3.3V to indicate a range of 0% to 100% relative humidity (RH) at standard temperature and pressure (STP). A simple feedback controller 20 receives the output from the sensor 12 and is used to trigger the droplet generator 2 if the voltage is below 1.8V (c.40%RH) and switch off the droplet generator 2 if the output is above 2.0V (c.50%RH). A simple on/off switch (not shown) is used to enable droplet generation to be initiated by the control circuit and a suitable LED (not shown) may be used to indicate completion of generation of the dose (which may, for example, be measured by the total integrated "on time" of the droplet generator 2). A solvent sensor of the pellistor type or combustible gas sensor may be used where the solvent is non-aqueous.

The outlet connecting tube 6, T-piece 8, one way valves 7, 10, filter 11 and mouthpiece 5 may, in some cases, be disposable items which are not reused.

By means of such apparatus, we have found that a dose of an active substance can be delivered to the lungs of a patient in a very effective manner, as a result of the small size of the particles/droplets which can be produced in the outlet of the chamber.

Being able to control the atmosphere within the chamber is an important aspect of the invention. To this end there are primarily three types of humidity control that have, so far, been used with the method and device of the invention in order to obtain a stable humidity in the chamber and thus to control the evaporation of the moisture from the droplets.

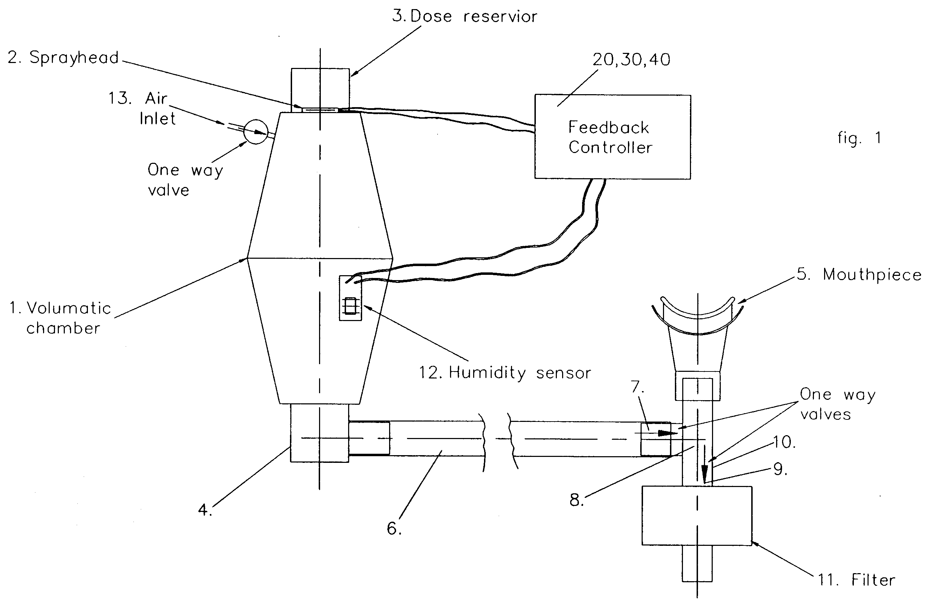

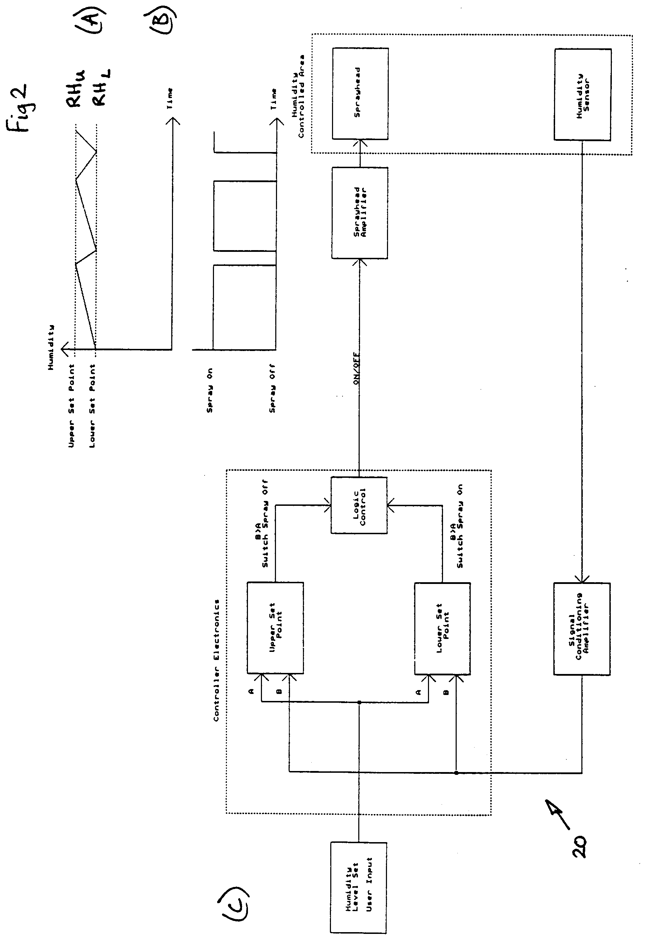

The first is a simple ON-OFF controller 20, an example of which is illustrated by reference to Figure 2, and which operates in a similar way to a simple thermostat controller. In this case a range of humidity $RH_L$ - $RH_U$ (see Figure 2A) is chosen for normal device operation. When the humidity is lower than the lower set point $RH_L$, the aerosol generator 2 is switched on to introduce the aerosol droplets into the control chamber 1. This ON-state continues until the humidity sensor 12 senses that the humidity in the chamber 1 has reached the upper limit $RH_U$, when the controller switches the aerosol generator 2 off to allow the chamber to vent (eg. by inhalation of air through the one-way valve mechanism 7 of Figure 1). The humidity in the controlled chamber 1 falls until it reaches the lower limit $RH_L$, when the controller 20 switches the aerosol generator back on.

This ON-OFF type of control is relatively crude, but it can be relatively easily implemented. However, the independence of flow rate from the droplet generator might cause significant humidity 'over-shoot', especially in smaller volume, high liquid flow-rate systems. This may lead to higher sensitivity to external atmospheric humidity which can be undesirable.

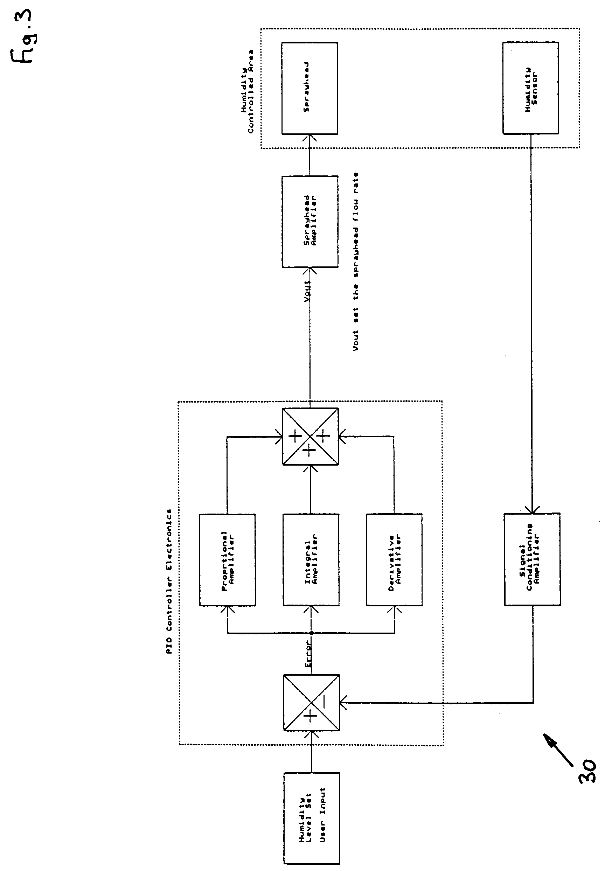

A second form of controller, illustrated in Figure 3, uses a well-known PID type (proportional, integral derivative) controller 30 to vary the drive voltage to the aerosol generator 2 in order to vary the liquid flow-rate delivered into the controlled chamber 1. In this case the aerosol generator 2 is operating continuously, but the controller uses the humidity sensor 12 and a pre-set target humidity to vary flow-rate into the chamber. This technique can be used to reduce the over-shoot problems of the ON-OFF controller of Figure 2.

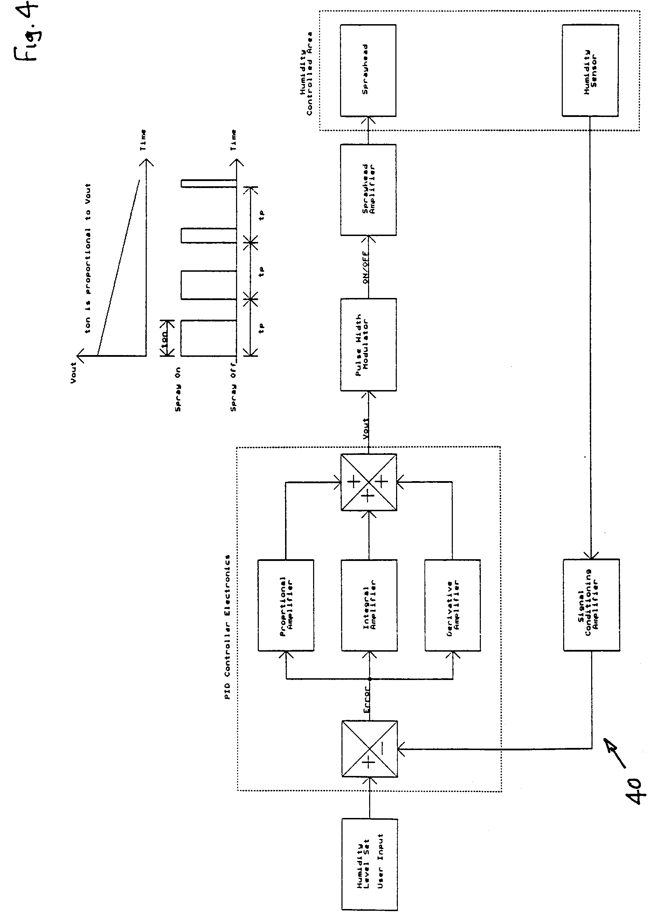

A third form of controller, illustrated in Figure 4, uses PID circuitry with pulse-width-modulation to vary the on-time $t_{on}$ of the aerosol generator 2 , which is operated at its optimal flow-rate. As the humidity reaches the pre-set value, the on-time is reduced to compensate. This operation is governed by a regular time interval $t_p$ (see Figure 4B) which is nominally fixed, and the on-time $t_{on}$ within each time interval $t_p$ is derived from the PID controller 40. This form of PID controller may be the most suitable to the humidity feedback controller for devices of the invention.

The single sensor described above measures the humidity of the chamber 1. However, if a second sensor is placed at the air inlet 13 then the two sensors can be used in a differential or ratiometric mode to enable the PID controller to take account of the ambient atmospheric conditions. This further helps to prevent over-shoot of the humidity within the chamber, and also acts as a warning if the ambient humidity is too for effective use in the device.

In this specification, the term 'humidity' is used to mean the relative humidity, ie. the ratio of partial vapour pressure $(p)$ of the liquid, from which an aerosol is to be generated, to the saturated vapour pressure $(p_s)$ of the liquid at the temperature and pressure within the controlled chamber; thus

$$Humidity = 100 \, \frac{p}{p_s}$$

Particular fields of use of the invention are presently considered to be:

- Drug inhalation for pulmonary or systemic therapy.
- Methods of examination (eg. by scintigraphy imaging) of lung volumes.

**Claims**

1. A method of generating an aerosol in which droplets are sprayed into an evaporation chamber to provide an aerosol, the chamber having an air or gas inlet and an outlet from which the aerosol is withdrawn, and in which the atmosphere within the chamber is controlled in order to control the extent of droplet evaporation within the chamber, in turn to control the size of the aerosol droplets, by sensing the atmosphere within the chamber and controlling the spraying of the droplets into the chamber in dependence upon the sensing.

2. A method of controlling the diameter of a liquid droplet aerosol within a chamber, the chamber having an air or gas inlet and outlet from which the aerosol exits the chamber, wherein the atmosphere within the chamber, the initial droplet diameter, and the droplet residence time are selected or controlled in order to control the extent of evaporation within the chamber, in turn to control the size of the aerosol droplets which exit the chamber.

3. A method according to claim 1 or claim 2, wherein the droplets are generated by an electronic aerosol generating device.

4. A method according to claim 3, wherein the electronic aerosol generating device is of the perforate membrane type, so as to create droplets having a narrow and small droplet diameter distribution.

5. A method according to claim 1, wherein the step of sensing the atmosphere comprises sensing the humidity within the chamber.

6. A method according to claim 1, wherein the step of sensing the atmosphere comprises sensing the level of solvent saturation within the chamber.

**7.** A method according to claim 1, further comprising sensing the atmosphere additionally at the air or gas inlet.

**8.** An aerosol generation apparatus for generating an aerosol of selected droplet size, comprising an evaporation chamber having an air or gas inlet and an outlet; an aerosol generator in use receiving liquid and generating an aerosol from that liquid in the evaporation chamber; an atmosphere sensor within the chamber; and means for controlling the aerosol generator in response to an output of the atmosphere sensor.

**9.** An aerosol generation apparatus according to claim 8, wherein the humidity sensor is a resistive or capacitive sensor.

**10.** An aerosol generation apparatus according to claim 9, further comprising a feedback controller adapted to operate such that when the sensor inside the evaporation chamber detects an upper threshold level of a selected range of humidity, the aerosol generator is switched off, the controller being adapted to switch the generator on when the humidity drops below the bottom threshold level of the selected range.

**11.** An aerosol generation apparatus according to claim 8, wherein the aerosol generator comprises an electronic aerosol generating device.

**12.** An aerosol generation apparatus according to claim 9 and claim 11, further comprising a PID feedback controller adapted to vary a drive voltage of the electronic aerosol generating device.

**13.** An aerosol generation apparatus according to claim 12, wherein the PID feedback controller is adapted to provide a pulse-width modulated output voltage to drive the electronic aerosol generating device.

**14.** An aerosol generation apparatus according to claim 11, wherein the aerosol generator is of the perforate membrane type, so as to create droplets having a narrow and small droplet diameter distribution.

**15.** An aerosol generation apparatus according to claim 11 or claim 14, wherein the aerosol generator is actuated by a piezoelectric device.

**16.** An aerosol generation apparatus according to claim 8, further comprising a second atmosphere sensor disposed at the air or gas inlet.

**17.** A drug inhalation device including an aerosol generation apparatus according to any of claims 8 to 15.

**18.** A scintigraphic imaging device including an aerosol generation apparatus according to any of claims 8 to 15.

3. Dose reservior

2. Sprayhead

13. Air Inlet

One way valve

20,30,40

Feedback Controller

fig. 1

1. Volumatic chamber

5. Mouthpiece

12. Humidity sensor

One way valves

7.

10.

9.

8.

4.

6.

11. Filter

EP 0 919 252 A1

Fig 2

Humidity

Upper Set Point ......... $RH_u$ (A)
Lower Set Point ......... $RH_L$

→ Time (B)

Spray On

Spray Off

→ Time

Controller Electronics

(C)

Humidity Controlled Area

A
B
Upper Set Point    B>A Switch Spray Off

Humidity Level Set User Input

Logic Control

ON/OFF

Sprayhead Amplifier

Sprayhead

A
B
Lower Set Point    B>A Switch Spray On

20

Signal Conditioning Amplifier

Humidity Sensor

EP 0 919 252 A1

Fig.3

Fig. 4

European Patent
Office

**EUROPEAN SEARCH REPORT**

Application Number

EP 97 30 9492

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | WO 96 13292 A (ARADIGM CORP)<br><br>* page 26, line 7 - page 29, line 5; figure 4 *<br>* page 40, line 1 - page 40, line 35 *<br>* page 50, line 17 - page 50, line 21 *<br>--- | 1-6,8,9, 11-15,17 | A61M15/00<br>B05B12/00 |
| A | US 4 674 490 A (FRANKEL HARRY ET AL)<br><br>* the whole document *<br>--- | 1,2,5,6, 8-10,12, 13 | |
| A | US 4 710 887 A (HO JIM Y W)<br><br>* the whole document *<br>--- | 1,2,5,6, 8,9,17 | |
| A | US 3 936 270 A (GUNTHER DONALD ALBERT)<br>* abstract *<br>--- | 1,2,8,10 | |
| A | US 5 139 016 A (WASER JOHN)<br>* abstract *<br>--- | 18 | TECHNICAL FIELDS SEARCHED (Int.Cl.6) |
| A | US 4 717 596 A (BARBEE STEVEN G ET AL)<br>* abstract; figure 1 *<br>----- | 1 | A61M<br>B05B |

The present search report has been drawn up for all claims

| Place of search | Date of completion of the search | Examiner |
|---|---|---|
| BERLIN | 25 March 1998 | Jameson, P |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03 82 (P04C01)